(19) Europäisches Patentamt
European Patent Office
Office européen des brevets

(11) **EP 3 218 064 B1**

(12) **EUROPÄISCHE PATENTSCHRIFT**

(45) Veröffentlichungstag und Bekanntmachung des Hinweises auf die Patenterteilung:
**08.07.2020   Patentblatt 2020/28**

(21) Anmeldenummer: **15771658.0**

(22) Anmeldetag: **02.10.2015**

(51) Int Cl.:
*A61Q 5/06* (2006.01)      *A61K 8/81* (2006.01)

(86) Internationale Anmeldenummer:
**PCT/EP2015/072829**

(87) Internationale Veröffentlichungsnummer:
**WO 2016/074852 (19.05.2016 Gazette 2016/20)**

(54) **KOSMETISCHE ZUSAMMENSETZUNG ZUR TEMPORÄREN HAARVERFORMUNG**

COSMETIC COMPOSITION FOR TEMPORARILY SHAPING HAIR

COMPOSITION COSMÉTIQUE POUR LA MISE EN FORME TEMPORAIRE DES CHEVEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.11.2014   DE 102014223088**

(43) Veröffentlichungstag der Anmeldung:
**20.09.2017   Patentblatt 2017/38**

(73) Patentinhaber: **Henkel AG & Co. KGaA**
**40589 Düsseldorf (DE)**

(72) Erfinder:
• **MARTINEZ, Cyrielle**
**22765 Hamburg (DE)**
• **BERMUDEZ AGUDELO, Maria Catalina**
**64295 Darmstadt (DE)**
• **LANGE, Julia Bibiane**
**24641 Sievershütten (DE)**
• **RICHTERS, Bernd**
**21129 Hamburg (DE)**

(56) Entgegenhaltungen:
EP-A1- 0 897 711          DE-A1-102009 001 978
DE-A1-102011 077 364          DE-A1-102011 088 818
DE-A1-102012 214 380          DE-A1-102013 225 753

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft eine kosmetische Zusammensetzung zur Haarfestigung bzw. zur temporären Umformung von keratinischen Fasern, insbesondere menschlichen Haaren, wobei die Zusammensetzung eine Kombination eines speziellen Acrylatcopolymers und eines Vinylpyrrolidon-Homopolymers enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurden als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

Bekannte anionische Polymere, die in Haarfestigungsprodukten Anwendung finden, sind vernetzte anionische amphiphile Polymere, die eine (Meth)acrylsäureeinheit und eine (Meth)acrylsäureoxyalkylenalkylestereinheit enthalten. Derartige Polymere sind bspw. in EP897711 B1, DE 102011077364 A1 und DE 102009001978 A1 beschrieben und sind im Handel z.B. unter der Bezeichnung Aculyn® 88 (INCI: Acrylates/Steareth-20 Methacrylate Crosspolymer) erhältlich. DE 10 2011 077 364 A1 und DE 10 2009 001 978 A1 betreffen die Verwendung eines solchen Polymers, speziell auch Aculyn® 88, in Kombination mit einem weiteren speziellen vernetzten anionischen Polymer zur temporären Umformung von Haaren.
Ein ähnliches Polymer ist im Handel unter der Bezeichnung BALANCE® RCF (INCI: Acrylates/Ceteareth-20 Methacrylate Crosspolymer) erhältlich, wobei dessen Funktion in Stylingprodukten im Wesentlichen die eines Verdickungsmittels und Filmbildners ist.

Aus der DE 102013225753 A1 sind kosmetische Mittel auf Grundlage einer spezifischen Polymerkombination bekannt. Insbesondere werden Kombinationen von Aculyn® 88 mit einem Galactomannan offenbart.

In der DE 102012214380 A1 werden kosmetische Zusammensetzungen auf Basis von hydrophob modifizierten (Meth)acrylsäure Copolymeren und hydrophob modifizierten Polysacchariden offenbart.

Die DE 102011088818 A1 beschreibt pulverförmige Zusammensetzungen, die festigende nichtionische Polymere wie Polyvinylpyrrolidon, und pulverförmige Verdickungsmittel wie Aculyn® 88 enthalten können.

Bekannte nichtionische Polymere, die in Haarfestigungsprodukten Anwendung finden, sind Polyvinylpyrrolidone (PVP). Es handelt sich dabei um Homopolymere von Vinylpyrrolidon. Vinylpyrrolidon-Homopolymere werden beispielsweise unter der Bezeichnung Luviskol® (BASF) vertrieben und werden üblicherweise als Filmbildner und/oder Verdickungsmittel eingesetzt. Insbesondere für die Verwendung in kosmetischen Zusammensetzungen und Stylingprodukten als Filmbildner oder Verdickungsmittel ist die Produktreihe Luviskol® K (BASF) erhältlich, in der Vinylpyrrolidon-Homopolymere in verschiedenen Molekulargewichten angeboten werden.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine bessere Kombination aus Steifheit (Stiffness) und Langzeithalt gewünscht wird. Es ist daher eine Aufgabe der vorliegenden Erfindung, derartige Stylingmittel bereitzustellen, die neben den oben genannten Eigenschaften insbesondere eine hervorragende Kombination der Eigenschaften Steifheit und Langzeithalt ergeben.

Dies wurde erfindungsgemäß durch eine Kombination eines bestimmten vernetzten anionischen Acrylatharzes und eines bestimmten nichtionischen Polymers erreicht.

Durch die vorliegende Erfindung wird bereitgestellt:
Eine kosmetische Zusammensetzung zur temporären Umformung keratinischer Fasern, welche enthält:

 (a) mindestens ein Vinylpyrrolidon-Homopolymer und

 (b) mindestens ein vernetztes Acrylatcopolymer, enthaltend ein vernetztes Acrylatcopolymer mit der INCI-Bezeichnung Acrylates/Ceteareth-20 Methacrylate Crosspolymer..

Kosmetische Zusammensetzung nach Punkt 1, wobei das Vinylpyrrolidon-Homopolymer (a) einen K-Wert (1 Gew.-%ige Lösung von PVP, Brookfield bei 23°C) in Wasser von 20 bis 100 hat.

Kosmetische Zusammensetzung Punkt 1 oder 2, wobei das Vinylpyrrolidon-Homopolymer (a) einen K-Wert von 80 bis 100, insbesondere etwa 90, hat.

Kosmetische Zusammensetzung einem der vorhergehenden Punkte, wobei die Zusammensetzung weiterhin mindestens ein Verdickungsmittel, insbesondere ein Carbomer, enthält.

Kosmetische Zusammensetzung nach einem der vorhergehenden Punkte, welche, bezogen auf das Gesamtgewicht der kosmetischen Zusammensetzung, enthält:

 0,01 bis 3,0 Gew.-% des Vinylpyrrolidon-Homopolymers (a) und
 0,01 bis 2,0 Gew.-% des Acrylatcopolymers (b).

Kosmetische Zusammensetzung nach einem der vorhergehenden Punkte, enthaltend, bezogen auf das Gesamtgewicht der kosmetischen Zusammensetzung:

 0,2 bis 2,0 Gew.-% des Vinylpyrrolidon-Homopolymers (a) und
 0,1 bis 1,2 Gew.-% des Acrylatcopolymers (b).

Kosmetische Zusammensetzung nach einem der Punkte 6 bis 9, enthaltend, bezogen auf das Gesamtgewicht der kosmetischen Zusammensetzung:
0,05 bis 1,5 Gew.-% des Carbomers.

Kosmetische Zusammensetzung nach einem der vorhergehenden Punkte, wobei die Zusammensetzung als Haargel, Haarspray, Haarwachs oder als Haarschaum vorliegt.

Es wurde im Rahmen der vorliegenden Erfindung überraschend festgestellt, dass durch Kombination zweier an sich bekannter Polymere eine deutlich verbesserte Kombination aus Langzeitwirkung und Steifheit von Stylingprodukten, insbesondere von Haargelen, erhalten werden kann, wobei die beiden notwendigen Komponenten offensichtlich synergistisch zusammenwirken. Eine derartige Kombination von guter Steifheit und gutem Langzeithalt war nicht erwartbar.

Der Begriff keratinische Fasern umfasst erfindungsgemäß Pelze, Wolle und Federn, insbesondere aber menschliche Haare.

Die wesentlichen Bestandteile der erfindungsgemäßen kosmetischen Zusammensetzung sind das Vinylpyrrolidon-Homopolymer (a) und das vernetzte Acrylatcopolymer (b).

Das erfindungsgemäße Mittel enthält als Komponente (a) zwingend ein Vinylpyrrolidon-Homopolymer. Es ist erfindungsgemäß bevorzugt, die Vinylpyrrolidon-Homopolymere auszuwählen aus Vinylpyrrolidon- Homopolymeren mit einem K-Wert (1 Gew.-%igen Lösung von PVP, Brookfield bei 23°C) in Wasser von 20 bis 100. Bevorzugter ist ein K-Wert von 80 bis 100, bevorzugter etwa 90. Der auch als Eigenviskosität bezeichnete K-Wert ist ein aus der relativen Viskosität mittels Viskositätsmessungen von Polymerlösungen einfach zu bestimmender Parameter zur Charakterisierung von Polymeren.

Bevorzugte Vinylpyrrolidon-Homopolymere sind erhältlich unter dem Handelsnamen Luviskol® K 30, Luviskol® K 80, Luviskol® K 85, Luviskol® K 90, jeweils von der Firma BASF SE. Am bevorzugtesten ist erfindungsgemäß Luviskol® K 90. Luviskol® K90 ist eine 20 % wässerige, farblose bis leicht gelbliche Lösung von Polyvinylpyrrolidon. Das Produkt hat einen K-Wert von 90,0 bis 98,0 (1% (m/V) in Wasser), einen Feststoffgehalt von 19.0 bis 21.0 Gew.-% und einen pH-Wert von 7,0 bis 9,0 (10 Gew.-% Feststoffgehalt in Wasser).

Das vernetzte anionische Acrylatcopolymer (b) enthält ein vernetztes Acrylatcopolymer mit der INCI-Bezeichnung Acrylates/Ceteareth-20 Methacrylate Crosspolymer.

Das vernetzte Acrylatcopolymer (b) ist aufgrund der enthaltenen Struktureinheiten amphiphil. Unter "amphiphil" versteht der Fachmann im Allgemeinen die Tatsache, dass ein und dasselbe Molekül hydrophile Strukturelemente (beispielsweise solche der Formeln (b-1)) und lipophile Strukturelemente (beispielsweise solche der Formel (b-2)) umfasst.

Die Vernetzung der vernetzen, amphiphilen, anionischen Polymere (b) kann bevorzugt durch Verwendung mindestens eines vernetzenden Monomers bewerkstelligt werden. Dabei ist es wiederum bevorzugt, die vernetzenden Monomere aus mindestens einer Verbindung der Gruppe zu wählen, die gebildet wird aus polyungesättigten aromatischen Monomeren (wie beispielsweise Divinylbenzol, Divinylnaphthalin, Trivinylbenzol), polyungesättigten alicyclischen Monomeren (wie beispielsweise 1,2,4-Trivinylcyclohexan), di-funktionellen Estern der Phthalsäure (wie beispielsweise Diallylphthalat), polyungesättigte aliphatische Monomere (wie beispielsweise Diene, Triene, Tetraene wie Isopren, 1,3-Butadien, 1,5-Hexadien, 1,5,9-Decatrien, 1,9-Decadien, 1,5-Heptadien), Polyalkenylether (wie beispielsweise Triallylpentaerythritol, Diallylpentaerythritol, Diallylsucrose, Octaallylsucrose, Trimethylolpropandiallylether), polyungesättigte Ester von Polyalkoholen oder Polysäuren (wie beispielsweise 1,6-Hexandioldi(meth)acrylat, Tetramethylentri(meth)acrylat, Allylacrylat, Diallylitaconat, Diallylfumarat, Diallylmaleat, Trimethylolpropantri(meth)acrylat, Trimethylolpropandi(meth)acrylat, Polyethyleneglycoldi(meth)acrylat), Alkylenebisacrylamide (wie beispielsweise Methylenbisacrylamid, Propylenbisacrylamid) Hydroxy- und Carboxyderivate des Methylenbisacrylamids (wie beispielsweise N,N'-Bismethylolmethylenbisacrylamid), Polyethyleneglycoldi(meth)acrylate (wie beispielsweise Ethyleneglycoldi(meth)acrylat, Diethyleneglycoldi(meth)acrylat, Triethyleneglycoldi(meth)acrylat), polyungesättigte Silane (wie beispielsweise Dimethyldivinylsilan, Methyltrivinylsilan, Allyldimethylvinylsilan, Diallyldimethylsilan, Tetravinylsilan), N-Methylolacrylamid; N-alkoxy(meth)acrylamid, wobei die Alkoxygruppe eine (C1 bis C18)-Alkoxygruppe ist, ungesättigte hydrolysierbare Silane (wie beispielsweise Triethoxyvinylsilan, Trisisopropoxyvinylsilan, 3-Triethoxysilylpropylmethacrylat), hydrolyierbare Silane (wie beispielsweise Ethyltriethoxysilan, Ethyltrimethoxysilan), Epoxy-substituierte hydrolysierbare Silane (wie beispielsweise 2-(3,4-Epoxycyclohexyl)ethyltriethoxysilan, 3-Glycidoxypropyltrimethyoxysilan) Polyisocyanate (wie beispielsweise 1,4-Diisocyanatobutan, 1,6-Diisocyanatohexan, 1,4-Phenylenediisocyanat, 4,4'-Oxybis(phenylisocyanat), ungesättigte Epoxide (wie beispielsweise Glycidylmethacrylate, Allylglycidylether), Polyepoxide (wie beispielsweise Diglycidylether, 1,2,5,6-Diepoxyhexan, Ethylenglycoldiglycidylether), ethoxylierte Polyole (wie beispielsweise Diole, Triole und Diphenole, jeweils ethoxyliert mit 2 bis 100 mol Ethyleneoxid pro Mol Hydroxylgruppen und terminiert mit einer polymerisierbaren ungesättigten Gruppe, wie beispielsweise Vinylether, Allylether, Acrylateester, Methacrylateester; Beispiele umfassen Bisphenol A ethoxyliertes di(meth)acrylat, Bisphenol F ethoxyliertes Di(meth)acrylat, ethoxyliertes Trimethylolpropantri(meth)acrylate, Acrylat- und Methacrylatester von Polyolen mit mindestens zwei Acrylatester- oder Methacrylatester-Funktionalitäten (wie beispielsweise Trimethylolpropantriacrylat (TMPTA), Trimethylolpropanethoxylated (15) triacrylat (TMPEO15TA), Trimethylolpropandimethacrylat, Triethyleneglycoldimethacrylat (TEGDMA), mit 30 Mol Ethylenoxid ethoxylliertes Bisphenol A-dimethacrylat (EOBDMA)).

Das vernetzte Acrylatcopolymer (b) mit der INCI-Bezeichnung Acrylates/Ceteareth-20 Methacrylate Crosspolymer ist ganz besonders bevorzugt eines, das unter dem Handelsnamen BALANCE® RCF (AkzoNobel) erhältlich ist. Bei letzterem handelt es sich um eine etwa 30 Gew.-% Dispersion in Wasser.

In einer besonders bevorzugten Ausführungsform der vorliegenden Erfindung enthält die kosmetische Zusammensetzung als das Vinylpyrrolidon-Homopolymer (a) das im Handel unter der Bezeichnung Luviskol® K90 (BASF) erhältliche Copolymer und als das anionische vernetzte Acrylatcopolymer (b) das im Handel unter der Bezeichnung BALANCE® RCF erhältliche Copolymer. Bei dieser Kombination wurden besonders gute Ergebnisse hinsichtlich einer Kombination von Steifheit und Langzeithalt in verschiedenen Konfektionierungen erzielt. Weitere allgemein geforderte Eigenschaften von Stylingprodukten, wie z. B. Feuchtebeständigkeit und niedrige Klebrigkeit, werden insbesondere mit dieser Kombination gleichfalls erzielt.

Die kosmetische Zusammensetzung der vorliegenden Erfindung enthält das Vinylpyrrolidon-Homopolymer (a) und das Acrylatcopolymer (b) in für Stylingmittel üblichen und geeigneten Mengen, die für die spezielle Anwendung und Konfektionierung angepasst werden können.

Die erfindungsgemäße kosmetische Zusammensetzung enthält das Vinylpyrrolidon-Homopolymer (a), bezogen auf das Gesamtgewicht der kosmetischen Zusammensetzung, z. B. in einer Menge von 0,01 bis 5,0 Gew.-%, bevorzugt 0,1 bis 3,0 Gew.-%, weiter bevorzugt von 0,2 bis 2,0 Gew.-%, weiterhin bevorzugt 0,5 bis 1,5 Gew.-% oder 0,8 bis 1,2 Gew.-%, jeweils angegeben als Feststoffgehalt aktiver Substanz in der kosmetischen Zusammensetzung.

Die erfindungsgemäße kosmetische Zusammensetzung enthält das vernetzte Acrylatcopolymer (b), bezogen auf das Gesamtgewicht der kosmetischen Zusammensetzung, z. B. in einer Menge von 0,01 bis 3,0 Gew.-%, bevorzugt 0,015 bis 1,5 Gew.-%, weiter bevorzugt von 0,1 bis 1,2 Gew.-%, weiterhin bevorzugt 0,2 bis 0,7 Gew.-%, jeweils angegeben als Feststoffgehalt aktiver Substanz in der kosmetischen Zusammensetzung.

Bevorzugt enthält die kosmetische Zusammensetzung der vorliegenden Erfindung eine oder mehrere weitere, als Verdickungsmittel oder Gelbildner wirkende Komponente(n), die von dem Vinylpyrrolidon-Homopolymer (a) und dem vernetzten Acrylatcopolymer (b) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Die vorliegende Erfindung umfasst aber auch Ausführungsformen, bei denen die kosmetische Zusammensetzung außer den Komponenten (a) und (b) keine weiteren als Verdickungsmittel, Filmbildner oder Gelbildner wirkenden Komponenten enthält.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Beispiele für nichtionische Polymere sind:

- Vinylpyrrolidon/Vinylester-Copolymere, wie sie beispielsweise unter dem Warenzeichen Luviskol(BASF) vertrieben werden. LuviskolVA 64 und LuviskolVA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminalund Benecel (AQUALON) vertrieben werden.
- Schellack.

- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Bevorzugt ist die weitere als Gelbildner wirkende Komponente eine Homopolyacrylsäure (INCI: Carbomer), die im Handel unter dem Namen Carbopol® in unterschiedlichen Ausführungen erhältlich ist. Das Carbomer ist bevorzugt in einem Anteil von 0,02 bis 3 Gew.-%, bevorzugt 0,05 bis 1,5 Gew.-% und noch bevorzugter 0,2 bis 0,8 Gew.-%, in Bezug auf das Gesamtgewicht der kosmetischen Zusammensetzung, enthalten. In Ausführungsfprmen der erfindung enthält die kosmetische Zusammensetzung neben den Komponenten (a) und (b) und dem Carbomer keine weiteren als Gelbildner, Filmbildner oder Verdickunsgmittel wirkenden Komponenten.

Die erfindungsgemäße kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von C6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäßen Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Zur Einstellung des pH kann die kosmetische Zusammensetzung der vorliegenden Erfindung weiterhin Neutralisatoren bzw. pH-Stellmittel enthalten. Beispiele von in Stylingprodukten verwendeten Neutralisatoren sind primäre Aminoalkohole. Ein Bespiel eines derartigen erfindungsgemäß bevorzugt verwendbaren Neutralisators ist Aminomethyl Propanol (INCI), das im Handel bspw. unter der Bezeichnung AMP-ULTRA® PC erhältlich ist. AMP-ULTRA® PC 2000 ist erfindungsgemäß bevorzugt und enthält 5% Wasser.

Die erfindungsgemäßen kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Es sind erfindungsgemäß die üblicherweise in kosmetischen Mittel eingesetzten Treibmittel verwendbar. Dimethylether ist erfindungsgemäß ein geeignetes Treibmittel.

Die vorliegende Erfindung betrifft auch die Verwendung von erfindungsgemäßen kosmetischen Zusammensetzungen zur temporären Umformung von keratinischen Fasern, insbesondere von menschlichen Haaren, sowie ein Verfahren zur temporären Verformung von keratinischen Fasern, insbesondere menschlichen Haaren, bei dem die erfindungsgemäße kosmetische Zusammensetzung auf keratinische Fasern appliziert wird.

Tabellarische Übersicht

Die Zusammensetzung einiger bevorzugter kosmetischer Mittel kann den folgenden Tabellen entnommen werden (Angaben in Gew.-% bezogen auf das Gesamtgewicht des kosmetischen Mittels, sofern nicht anders angegeben).

| | Formel 1 | Formel 2 | Formel 3 | Formel 4 | Formel 5 |
|---|---|---|---|---|---|
| Vinylpyrrolidon-Homopolymer (a) | 0,05 bis 3,0 | 0,1 bis 2,5 | 0,2 bis 2,0 | 0,5 bis 1,5 | 0,8 bis 1,2 |
| Copolymer (b), enthaltend Acrylates/ Ceteareth-20 Methacrylate Crosspolymer | 0,01 bis 5,0 | 0,15 bis 4,0 | 0,1 bis 2,0 | 0,5 bis 1,5 | 0,2 bis 0,7 |
| Misc | add 100 | add 100 | add 100 | add 100 | add 100 |

| | Formel 1a | Formel 2a | Formel 3a | Formel 4a | Formel 5a |
|---|---|---|---|---|---|
| Vinylpyrrolidon-Homopolymer (a) mit K-Wert von 80 bis 100 (angegeben als Feststoffgehalt) | 0,05 bis 3,0 | 0,1 bis 2,5 | 0,2 bis 2,0 | 0,5 bis 1,5 | 0,8 bis 1,2 |
| Copolymer b): Acrylates/Ceteareth-20 Methacrylate Crosspolymer | 0,01 bis 5,0 | 0,15 bis 4,0 | 0,1 bis 2,0 | 0,5 bis 1,5 | 0,2 bis 0,7 |
| Misc | add 100 | add 100 | add 100 | add 100 | add 100 |

| | Formel 1b | Formel 2b | Formel 3b | Formel 4b | Formel 5b |
|---|---|---|---|---|---|
| Vinylpyrrolidon-Homopolymer (a): Luviskol® K 90 - 20% (angegeben als Feststoffgehalt) | 0,05 bis 3,0 | 0,1 bis 2,5 | 0,2 bis 2,0 | 0,5 bis 1,5 | 0,8 bis 1,2 |

(fortgesetzt)

| | Formel 1b | Formel 2b | Formel 3b | Formel 4b | Formel 5b |
|---|---|---|---|---|---|
| Copolymer b): BALANCE® RCF (angegeben als Feststoffgehalt) | 0,01 bis 5,0 | 0,15 bis 4,0 | 0,1 bis 2,0 | 0,5 bis 1,5 | 0,2 bis 0,7 |
| Misc | add 100 | add 100 | add 100 | add 100 | add 100 |

| | Formel 11 | Formel 12 | Formel 13 | Formel 14 | Formel 15 |
|---|---|---|---|---|---|
| Vinylpyrrolidon-Homopolymer (a) | 0,05 bis 3,0 | 0,1 bis 2,5 | 0,2 bis 2,0 | 0,5 bis 1,5 | 0,8 bis 1,2 |
| Copolymer b), enthaltend Acrylates/ Ceteareth-20 Methacrylate Crosspolymer | 0,01 bis 5,0 | 0,15 bis 4,0 | 0,1 bis 2,0 | 0,5 bis 1,5 | 0,2 bis 0,7 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |

| | Formel 11a | Formel 12a | Formel 13a | Formel 14a | Formel 15a |
|---|---|---|---|---|---|
| Vinylpyrrolidon-Homopolymer (a) mit K-Wert von 80 bis 100 (angegeben als Feststoffgehalt) | 0,05 bis 3,0 | 0,1 bis 2,5 | 0,2 bis 2,0 | 0,5 bis 1,5 | 0,8 bis 1,2 |
| Copolymer b): Acrylates/Ceteareth-20 Methacrylate Crosspolymer | 0,01 bis 5,0 | 0,15 bis 4,0 | 0,1 bis 2,0 | 0,5 bis 1,5 | 0,2 bis 0,7 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |

| | Formel 11b | Formel 12b | Formel 13b | Formel 14b | Formel 15b |
|---|---|---|---|---|---|
| Vinylpyrrolidon-Homopolymer (a): Luviskol® K 90 - 20% (angegeben als Feststoffgehalt) | 0,05 bis 3,0 | 0,1 bis 2,5 | 0,2 bis 2,0 | 0,5 bis 1,5 | 0,8 bis 1,2 |
| Copolymer b): BALANCE® RCF (Angaben als Feststoffgehalt) | 0,01 bis 5,0 | 0,15 bis 4,0 | 0,1 bis 2,0 | 0,5 bis 1,5 | 0,2 bis 0,7 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |

Unter "Misc" ist erfindungsgemäß ein kosmetischer Träger zu verstehen, insbesondere Wasser und ggf. weitere übliche Bestandteile von Stylingprodukten.

Beispiele

1. Es wurde folgendes Stylinggel hergestellt:

| Zusammensetzung | 1 | | |
|---|---|---|---|
| Komponente/Rohstoff | Gew.-% | INCI-Bezeichnung | Hersteller |
| Water, demineralized, without $H_2O_2$ | 93,320 | | |
| Carbomer 20000-30000 mPas (0,2 %) | 0,505 | Carbomer | |
| Luviskol® K90 - 20 % | 5,000 | PVP | BASF |
| BALANCE® RCF | 1,665 | Acrylates Copolymer (and) Water | AkzoNobel |
| AMP-ULTRA® PC 2000 | 0,510 | PEG-40 Hydrogenated Castor Oil | Dow Chemical |
| Gesamt | 100 | | |

Die Mengenangaben in der Tabelle sind in Gew.-% des jeweiligen Rohstoffs, bezogen auf die gesamte Zusammensetzung, angegeben.

Steifheit:

In eine trockene Haarsträhne (Euro-Naturhaar der Firma Kerling, 826500 Klebetresse dicht, einseitig geklebt, Gesamtlänge 150 mm, freie Länge 130 mm, Breite 20 mm, Gewicht 1,8 ± 0,2 g) wurden 850 mg einer gelförmigen Testzusammensetzung mit den Fingern einmassiert. Die mit der zu untersuchenden Testzusammensetzung behandelte Haarsträhne wird in einer Teflon-Schiene mit einem Durchmesser von 20 mm begradigt. Die präparierten Strähnen wurden anschließend über Nacht bei 21°C und 50% relativer Luftfeuchte im Klimaraum getrocknet und konditioniert.

Die konditionierte Strähne wurde vorsichtig aus der Teflon-Schiene genommen. Die entstandene flache Strähne wurde auf die 40mm voneinander entfernten Messblöcke gelegt. Darüber wird mittig der 3PB Adapter eines Universalprüfgeräts AMETEK LF Plus der Firma AMETEK Precision Instruments Europe GmbH, Produktgruppe Lloyd montiert. Die gesamte Messung erfolgte im Klimaraum unter konstanten klimatischen Bedingungen bei 21°C und 50% relativer Luftfeuchte.

Um standardisierte Ausgangsbedingungen zu schaffen, startete die Messung mit dem Anfahren einer Vorlast von 0,05 N. Anschließend wurde die Strähne mit einer Geschwindigkeit von 500 mm min$^{-1}$ um 15 mm gedrückt, wobei die dazu nötige Kraft gemessen wird. Nachdem die charakteristische Kraft K bei der maximalen Deformation von 15 mm aufgezeichnet wurde.

Mit dieser Messmethode lässt sich anhand der Kraft Fmax als Parameter der Steifheit des Polymerfilms und der Haltegrad des dadurch generierten Frisurenhalts messen.

Pro Testzusammensetzungen wurden 10 Strähnen erstellt und vermessen. Es wurden Steifheitswerte im Bereich von 3 -8 N erhalten, wobei es sich um arithmetische Mittel handelte.

Langzeithalt:

Die Zusammensetzung wurde mittels einer Long Lasting Hold Messung hinsichtlich ihrer formgebenden Eigenschaften überprüft. Hierzu wurden standardisierte Haarsträhnen der Fa. Kerling (Art. Nr. 826500) des Haartyps "European Natural, Farbe 6/0" von einer Länge ($L_{max}$) von 220 mm und einem Gewicht von 3,0g eingesetzt. Zur Vorbereitung wurden die Strähnen mit einer 12,5 Gew.-%igen Natriumlaurethsulfat-Lösung gewaschen. Die Haarsträhnen wurden über Nacht in einem Trockenofen bei 318 K getrocknet.

Die Haare wurden 20 min in lauwarmen Wasser eingeweicht und dann auf ca. 50% Restfeuchte im Haare abgetupft,

750 mg der Zusammensetzung wurden auf jede der Haarsträhnen aufgebracht und einmassiert. Die Haarsträhnen wurden in eine Teflonschiene eingelegt, mittels einer Stahlrolle geglättet und über Nacht bei 21°C und 50% getrocknet.

Die Haarsträhnen wurden nachfolgend an ihrem eine Ende in eine Haltevorrichtung eingespannt, und für eine Dauer von sechs Stunden bei 21°C und 85% relativer Luftfeuchte gelagert. Zur Berechnung des Long Lasting Hold (LLH) wurden die aus der Haltevorrichtung herausragende Strähnenlänge vor ($L_0$) und nach ($L_t$) der Lagerung gemessen.

Der Long Lasting Hold ist ein Maß für die zeitliche Längenänderung einer mittels eines Haarverformungsmittels fixierten Haarsträhne. Je höher der LLH-Wert, desto geringer die Längenänderung der Haarsträhne unter Einfluss von Luftfeuchtigkeit in einer bestimmten Zeitdauer und desto besser der Haltegrad des Haarverformungsmittels.

Der Long Lasting Hold wurde nach folgender Formel berechnet.

$$LLH = 1 - (L_t - L_0/ L_{max})$$

Es wurde ein LLH-Werte von 40-60% ermittelt (arithmetisches Mittel der LLH-Werte von zehn Teststrähnen).

Das erfindungsgemäße Gel zeigte somit eine außergewöhnlich gute Kombination aus Langzeithalt und Steifheit.

**Patentansprüche**

1. Kosmetische Zusammensetzung zur temporären Umformung keratinischer Fasern, welche enthält:

   (a) mindestens ein Vinylpyrrolidon-Homopolymer und
   (b) mindestens ein vernetztes Acrylatcopolymer, enthaltend ein vernetztes Acrylatcopolymer mit der INCI-Bezeichnung Acrylates/Ceteareth-20 Methacrylate Crosspolymer.

2. Kosmetische Zusammensetzung nach Anspruch 1, wobei das Vinylpyrrolidon-Homopolymer (a) einen K-Wert (1 Gew.-%ige Lösung von PVP, Brookfield bei 23°C) in Wasser von 20 bis 100 hat.

3. Kosmetische Zusammensetzung Anspruch 1 oder 2, wobei das Vinylpyrrolidon-Homopolymer (a) einen K-Wert von 80 bis 100, insbesondere etwa 90, hat.

4. Kosmetische Zusammensetzung einem der vorhergehenden Ansprüche, wobei die Zusammensetzung weiterhin mindestens ein Verdickungsmittel, insbesondere ein Carbomer, enthält.

5. Kosmetische Zusammensetzung nach einem der vorhergehenden Ansprüche, welche, bezogen auf das Gesamtgewicht der kosmetischen Zusammensetzung, enthält:

   0,01 bis 3,0 Gew.-% des Vinylpyrrolidon-Homopolymers (a) und
   0,01 bis 2,0 Gew.-% des Acrylatcopolymers (b).

6. Kosmetische Zusammensetzung nach einem der vorhergehenden Ansprüche, enthaltend, bezogen auf das Gesamtgewicht der kosmetischen Zusammensetzung:

   0,2 bis 2,0 Gew.-% des Vinylpyrrolidon-Homopolymers (a) und
   0,1 bis 1,2 Gew.-% des Acrylatcopolymers (b).

7. Kosmetische Zusammensetzung nach einem der Ansprüche 4 bis 6, enthaltend, bezogen auf das Gesamtgewicht der kosmetischen Zusammensetzung:
   0,05 bis 1,5 Gew.-% des Carbomers.

8. Kosmetische Zusammensetzung nach einem der vorhergehenden Ansprüche, wobei die Zusammensetzung als Haargel, Haarspray, Haarwachs oder als Haarschaum vorliegt.

**Claims**

1. A cosmetic composition for temporarily shaping keratin fibers, which contains:

   (a) at least one vinylpyrrolidone homopolymer and
   (b) at least one cross-linked acrylate copolymer, containing a cross-linked acrylate copolymer having the INCI name acrylates/ceteareth-20 methacrylate crosspolymer.

2. The cosmetic composition according to claim 1, wherein the vinylpyrrolidone homopolymer (a) has a K-value (1 wt.% solution of PVP, Brookfield at 23 °C) in water of 20 to 100.

3. The cosmetic composition according to claim 1 or 2, wherein the vinylpyrrolidone homopolymer (a) has a K-value of 80 to 100, in particular approximately 90.

4. The cosmetic composition according to one of the preceding claims, wherein the composition further contains at least one thickener, in particular a carbomer.

5. The cosmetic composition according to one of the preceding claims, containing, based on the total weight of the cosmetic composition:
0.01 to 3.0 wt.% of the vinylpyrrolidone homopolymer (a) and 0.01 to 2.0 wt.% of the acrylate copolymer (b).

6. The cosmetic composition according to one of the preceding claims, containing, based on the total weight of the cosmetic composition:
0.2 to 2.0 wt.% of the vinylpyrrolidone homopolymer (a) and 0.1 to 1.2 wt.% of the acrylate copolymer (b).

7. The cosmetic composition according to one of claims 4 to 6, containing, based on the total weight of the cosmetic composition:
0.05 to 1.5 wt.% of the carbomer.

8. The cosmetic composition according to one of the preceding claims, wherein the composition is in the form of a hair gel, hair spray, hair wax or hair foam.


**Revendications**

1. Composition cosmétique pour la mise en forme temporaire de fibres kératiniques, contenant :

   (a) au moins un homopolymère de vinylpyrrolidone, et
   (b) au moins un copolymère d'acrylate réticulé contenant un copolymère d'acrylate réticulé de dénomination INCI « Acrylates/Ceteareth-20 Methacrylate Crosspolymer ».

2. Composition cosmétique selon la revendication 1, l'homopolymère de vinylpyrrolidone (a) présentant une valeur K (solution à 1 % en poids de PVP, Brookfield à 23 °C) dans l'eau de 20 à 100.

3. Composition cosmétique selon la revendication 1 ou 2, l'homopolymère de vinylpyrrolidone (a) présentant une valeur K de 80 à 100, en particulier d'environ 90.

4. Composition cosmétique selon l'une des revendications précédentes, la composition contenant en outre au moins un épaississant, en particulier un carbomère.

5. Composition cosmétique selon l'une des revendications précédentes, laquelle contient, par rapport au poids total de la composition cosmétique :
0,01 à 3,0 % en poids de l'homopolymère de vinylpyrrolidone (a) et 0,01 à 2,0 % en poids du copolymère d'acrylate (b).

6. Composition cosmétique selon l'une des revendications précédentes, contenant, par rapport au poids total de la composition cosmétique :
0,2 à 2,0 % en poids de l'homopolymère de vinylpyrrolidone (a) et 0,1 à 1,2 % en poids du copolymère d'acrylate (b).

7. Composition cosmétique selon l'une des revendications 4 à 6, contenant, par rapport au poids total de la composition cosmétique :
0,05 à 1,5 % en poids du carbomère.

8. Composition cosmétique selon l'une des revendications précédentes, la composition se présentant sous la forme d'un gel capillaire, d'une laque capillaire, d'une cire capillaire ou d'une mousse capillaire.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 897711 B1
- DE 102011077364 A1
- DE 102009001978 A1
- DE 102013225753 A1
- DE 102012214380 A1
- DE 102011088818 A1